# EUROPEAN PATENT APPLICATION

(11) **EP 1 826 276 A1**
(43) Date of publication of application: **29.08.2007**
(21) Application number: 07290214.1
(22) Date of filing: 20.02.2007
(51) Int. Cl.: C12Q 1/04, C12Q 1/06, C12Q 1/66

(54) **Fast microbiological analysis method**

(30) Priority: 24.02.2006 FR 0650645
(71) Applicant: MILLIPORE CORPORATION, Billerica Massachusetts 01821 (US)
(72) Inventor: Grinon, Raphaël, 68150 Ribeauville (FR); Clauss, Christian, 67210 Obernai (FR); Pressel, Marie, 67120 Molsheim (FR); Ribault, Sébastian, 67380 Lingolsheim (FR)
(74) Representative: Santarelli

(57) **Abstract**

The method includes the step of selecting a reagent adapted to reveal the presence of ATP by luminescence, the step of depositing said reagent on a support adapted to retain microorganisms and the step of detecting the response of said support to said reagent; said detection step includes the step (90) of recording the quantity of light emitted by said support as a function of time from a time t₁ before a time t₀ at which said deposition step is carried out to a time t₂ after said time to, the step (91) of extrapolating, from said recorded quantity of light, to the quantity of light as a function of time that would have been emitted by said support without the deposition of said reagent, and the step (92) of determining from said recorded quantity of light and from said extrapolated quantity of light a set of values representative of the quantity of light emitted only as a result of bringing said reagent into contact with the ATP.

## Description

The present invention relates to a fast microbiological analysis method.

At present, checking the microbiological quality of liquids, gases or surfaces in the context of industrial and medical activities has to conform to strict standards.

Because of this, industry and health and safety authorities must have access to tools for detecting microbiological contamination as quickly as possible in order to be able to apply remedies in good time and at least cost.

In practice, microbiological monitoring is carried out on a gel medium on which microorganisms that have been collected on a microporous membrane are cultured until they become visible to the naked eye.

Incubation times vary from one microorganism to another but are generally at least 24 hours and sometimes longer for slower growing microorganisms (for example mycobacteria) or because the microorganisms have been stressed by their environmental conditions.

One way to speed up detection is to reduce the minimum culture time (or even to eliminate it completely in the case of certain microorganisms) by basing the detection of microorganisms on their metabolic activity.

A universal metabolic marker, usually adenosine triphosphate (ATP), contained in living microorganisms is measured by bringing it into contact with a bioluminescence reagent for revealing the presence of ATP by luminescence, so that the presence of microorganisms can be detected without having to wait for colonies to form on a gel medium and to become visible to the naked eye.

The quantity of light emitted is a function of the mass of ATP and therefore of the number of microorganisms.

A method for counting the number of living microorganisms in a test solution is already known, in particular from European patent 0 529 084, this method comprising the steps of:
- filtering the solution through a microporous membrane in order to retain the living microorganisms contained in the solution;
- spraying an agent adapted to render the ATP of the microorganisms accessible to a reagent that is sprayed afterwards and reveals the presence of ATP by luminescence;
- then spraying that reagent onto the membrane; and
- detecting the presence of microorganisms from the quantity of light emitted in response to the deposition of the reagent.

The quantity of light measured in this way comes on the one hand from the light emitted in response to the chemical reaction of the reagent with the ATP and on the other hand from "background light" emitted by the consumable independently of the presence or absence of microorganisms.

This kind of background light has diverse sources, for example the emission of natural light from the materials (by phosphorescence, fluorescence or thermal radiation).

To determine the presence of microorganisms on a membrane reliably, it is therefore necessary to distinguish light coming only from contact of the reagent with the ATP from "background light".

One solution is to establish empirically a discrimination threshold beyond which the light emitted is considered not to be caused by background noise alone but by the presence of microorganisms.

For this purpose series of sterile membranes are subjected to the treatment and then to the analysis of the light emitted in response to that treatment.

For each series, if the result of the analysis indicates erroneously the presence of microorganisms (these results are known as "false positives") on one or more sterile membranes, then the threshold is adjusted accordingly, until no false positives are detected for a series of membranes.

The invention aims to increase the reliability of the results of such analysis at the same time as remaining simple and convenient to use.

To this end it proposes a method of fast microbiological analysis of a support adapted to retain microorganisms, including the step of selecting a reagent adapted to reveal the presence of ATP by luminescence, and:
- the step of acting on said microorganisms to render the ATP of said microorganisms accessible to said reagent; then
- the step of depositing on said support said reagent; and
- the step of detecting the response of said support to said reagent;
characterized in that said detection step includes:
- the step of recording the quantity of light emitted by said support as a function of time from a time t₁ before a time t₀ at which said deposition step is carried out to a time t₂ after said time t₀;
- the step of extrapolating, from said recorded quantity of light, to the quantity of light as a function of time that would have been emitted by said support between said time t₁ and said time t₂ without the deposition of said reagent; and
- the step of determining from said recorded quantity of light and from said extrapolated quantity of light a set of values representative of the quantity of light emitted as a function of time starting from said time t₀ only as a result of bringing said reagent into contact with the ATP.

In the method of the invention, only one measurement (on the support to be analyzed) is necessary to isolate the wanted signal. Background light is extrapolated from this measurement by calculation without additional measurements having to be effected on the same support or on other supports.

Moreover, the background light is determined for each new measurement on a new support to be analyzed in order to update the data for each new support.

The method of the invention therefore determines the quantity of light emitted only because of the contact of the reagent with the ATP conveniently, with a single measurement and with increased accuracy.

According to features of the invention preferred for reasons of simplicity and convenience both of manufacture and of use:
- said step of determining said set of values includes the step of determining the difference between said recorded quantity of light and said extrapolated quantity of light;
- after the step of determining said set of values, the detection step further includes:
   - the step of integrating said set of values as a function of time; and
   - the step of comparing the result of that integration to a predetermined value to deduce the presence of microorganisms; and/or
- after the step of determining said set of values, the detection step further comprises:
   - the step of selecting the maximum value of said set of values;
   - the step of comparing the result of that selection to a predetermined value to deduce the presence of microorganisms;
According to other preferred features, to increase still further the reliability of the result, the detection step further comprises a step of discriminating false positives comprising a step of implementing at least one of the following tests:
- verifying that said quantity of light emitted by said support after the time t₀ decreases over time;
- verifying that the amplitude of the abrupt transition of light caused by the deposition of the reagent is greater than a predetermined value;
- verifying that the quantity of light remains greater than a predetermined value at a predetermined time after said deposition of the reagent with respect to the quantity of light before the deposition of the reagent;
- verifying that the decrease in the quantity of light emitted by said support after said deposition of the reagent is different from the decrease in the quantity of light emitted by said support before said deposition, and/or
- verifying that the time during which the quantity of light emitted by said support is greater than a predetermined threshold is greater than a predetermined time;
then after the step of implementing at least one of the preceding tests, the step of indicating the presence of a false positive if the result of at least one of the tests is negative.

This discrimination step makes it possible to ensure that the result obtained is not caused by an extraneous phenomenon but is indeed due to the presence of microorganisms on the support.

According to still other preferred features, for the same reasons as those stated above:
- the recording step includes:
   - the step of selecting a photomultiplier;
   - the step of placing said support facing said photomultiplier;
   - the step of opening at said time t₁ a closure member disposed in front of said photomultiplier;
   - the step of measuring the quantity of light received by said photomultiplier between said time t₁ and said time t₂; and
   - the step of storing said measurement in a memory;
- said extrapolation step is effected by calculating the coefficients of a logarithmic function decreasing as a function of time and varying as the quantity of light that would be emitted by said support without the deposition of said reagent would vary; and/or
- said extrapolation step is based on said quantity of light recorded between said time t₁ and said time t₀.

According to other features of the invention preferred for the same reasons as indicated hereinabove:
- said extrapolation step is based on said quantity of light recorded between a time t₀+x and said time t₂, where x is a predetermined duration.

The duration x is determined so that beyond said time t₀+x the influence of depositing the reagent on the quantity of light emitted by the support is negligible.

According to other features of the invention preferred for the same reasons as indicated hereinabove:
- said reagent for revealing the presence of ATP by luminescence is a reagent based on luciferin-luciferase;
- said step of acting on said microorganisms includes a step of lysis of said microorganisms;
- said lysis step is effected by heating said support;
- said heating step includes the step of raising the heating temperature to a value from 50°C to 150°C;
- said heating step includes the step of raising the heating temperature to a value from 70°C to 100°C;
- said support is heated by microwaves;
- said step of acting on said microorganisms includes the step of rendering said microorganisms permeable;
- said step of rendering said microorganisms permeable includes the step of spraying onto said support a reagent adapted to render said microorganisms permeable; and/or
- said support is a microporous membrane.

The features and advantages of the invention will emerge from the following description, which is given by way of preferred but nonlimiting example and with reference to the appended drawings, in which:
- figure 1 is a perspective view of a fast microbiological analysis device showing a mobile carriage of the device in a position in which it receives a filter unit;
- figure 2 is a perspective view to a larger scale than figure 1 of this device in section on a vertical plane centered on the path of the carriage;
- figures 3 and 4 are two views similar to figure 2 but respectively showing the mobile carriage at first and second positions for processing a membrane of the filter unit;
- figures 5 and 6 are two perspective views in section at different angles and to a larger scale than figure 3;
- figure 7 is a timing diagram showing, with a common time scale (the abscissa axis) and a common percentage scale (the ordinate axis), how, when the membrane that includes the filtration unit is heated to a temperature of 90°C, the rate of remanent activity of a reagent previously deposited onto the membrane and the rate of lysis of first and second types of microorganism present on the membrane vary as a function of time;
- figure 8 is a timing diagram showing, with a common time scale (the abscissa axis), the relative quantity of light emitted by the membrane and its immediate surroundings before, during and after the deposition of a reagent for revealing the presence of ATP by luminescence and the quantity of light that would have been emitted without depositing the reagent;
- figure 9 is a timing diagram similar to those of figure 8 but showing in isolation the quantity of light emitted only as a result of bringing the reagent for revealing the presence of ATP by luminescence into contact with the microorganisms on the membrane;
- figure 10 is a block diagram of the device, showing in particular a microcomputer of the device;
- figure 11 is a flowchart showing the operations effected by the microcomputer on data supplied by a photomultiplier of the device; and
- figure 12 is a timing diagram showing, with a common time scale (the abscissa axis), how the quantity of light emitted by the membrane and its immediate surroundings varies, for different initial amounts of unwanted ATP, before, during and after the deposition of the reagent for revealing the presence of ATP by luminescence.

The rapid analysis device 1 of a filter unit 15 includes a sprayer station 2, a lysis station 3 and a light measurement station 4.

The spraying station 2 and the measuring station 4 face each other and the lysis station 3 is in the vicinity of the stations 2 and 4.

The spraying station 2 includes a spraying nozzle 21 connected by a pipe 22 to a flask, not shown.

The spraying nozzle 21 has a conical head 25 and is fixed by means of a plate 23 to a frame of the device (not shown) .

The flask to which the pipe 22 is connected contains a reagent for revealing the presence of ATP by luminescence based on water, a luciferin-luciferase complex and magnesium. The flask carries an RFID chip holding information relating to the reagent, such as the initial volume of reagent contained in the flask, the expiry date of the reagent or the number of cycles that can be carried out using the remaining volume of reagent in the flask.

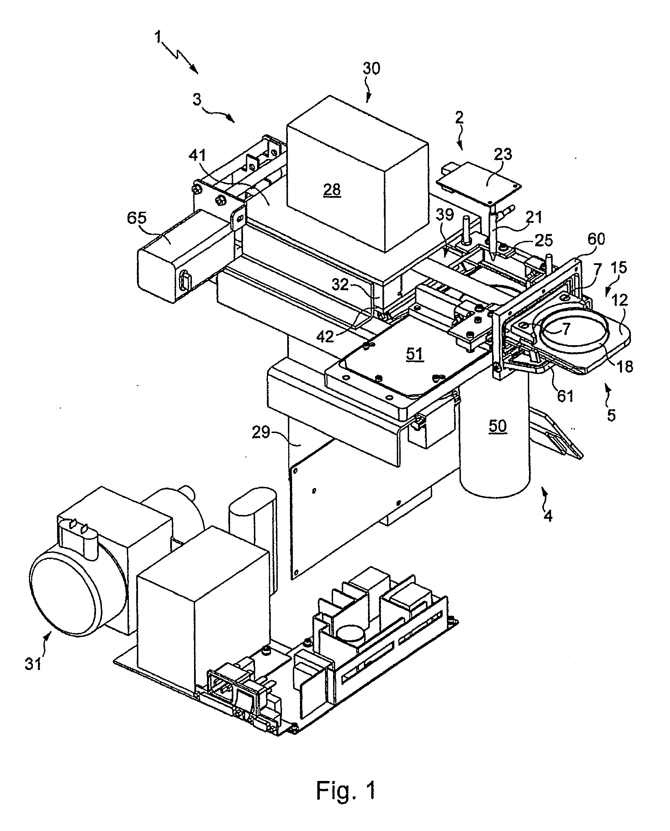

The lysis station 3 includes an enclosure 30 of generally parallelepipedal shape and a magnetron 31 (represented in figure 1 with its electronic control panel) connected by a coaxial cable (not shown) to an antenna (not visible) inside the enclosure 30.

Like the plate 23 of the nozzle 21, the magnetron 31 and its control panel are mounted on the frame of the device (not shown).

The enclosure 30 has an upper enclosure body 28, a lower enclosure body 29 and a U-section clamp 32.

Each enclosure body is of generally parallelepipedal shape and has an open face.

The enclosure body 28 (respectively 29) has around its open face a rectangular contour flange 41 (respectively 42) connected transversely to the remainder of the enclosure body.

The U-shaped clamp 32 has two large branches 43 connected together by a transverse small branch 45.

In the assembled state, each flange 41, 42 cooperates with the branches of the clamp 32 so that the open faces of the enclosure bodies 28 and 29 are disposed face to face.

The enclosure bodies 28, 29 and the clamp 32 of the enclosure 30 have dimensions such that the magnetron 31 produces standing waves in the cavity delimited by the enclosure 30.

In the assembled state, the space on the side of the enclosure opposite the branch 45, between the branches 43 and between the flanges 41 and 42 is open in the direction towards the spraying station 2 and the measuring station 4 by virtue of a window 39.

In the vicinity of the window 39 there is a flap 68 for blocking off this window to isolate the spraying station 2 and the measuring station 4 from electromagnetic interference generated by the magnetron 31.

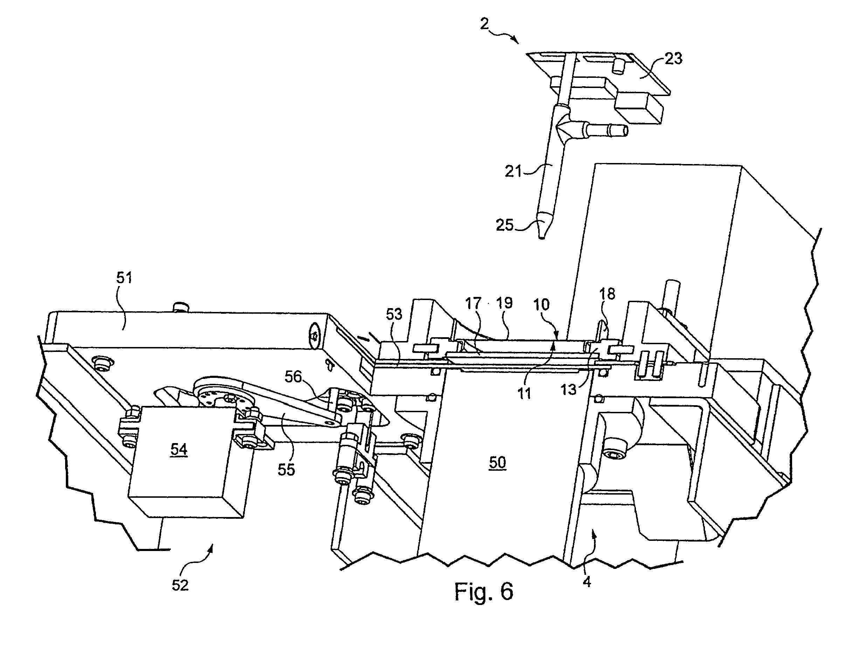

The measuring station 4 includes a photomultiplier 50 passing through a table 51 and a closure member 52 (figure 6).

The photomultiplier 50 is an Electron Tubes^{®} 9266B photomultiplier.

The closure member 52 is shown in detail in figure 6 and includes an opaque plate 53, a rod 56, a crank 55 and a motor 54.

The plate 53 is mobile and accommodated in a cavity formed in the table 51 and connected by the rod 56 to the crank 55.

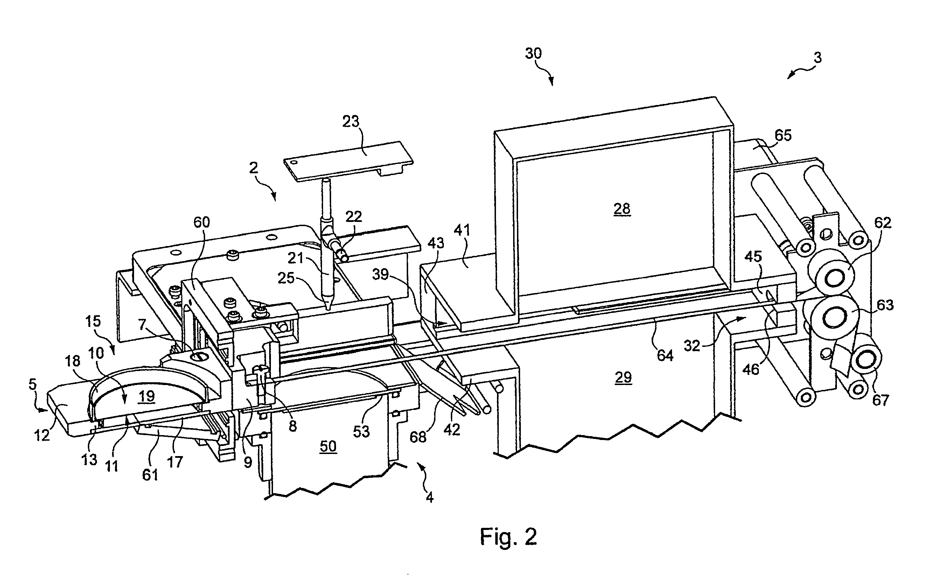

The device 1 also includes a carriage 5 having a plate 12 and a rim 9 (figures 2 to 4). The plate 12 is fixed to the rim 9 by two screws 7 (figure 1).

A cylindrical orifice in the plate 12 opens onto each side of the plate.

This orifice is flanked by an annular flange 13 recessed relative to each side of the plate 12.

The recess on the side of the plate 12 that faces the photomultiplier 50 houses a glass window 17.

The filter unit 15 includes a body 18 around a microporous membrane 19 having two opposite faces 10 and 11.

The rim 9 on the carriage 5 is connected by a belt 64 fixed to the rim by a screw 8 and by a set of pulleys 62, 63 and 67 to a motor 65 (figures 1 and 2) situated outside the enclosure 30 in the vicinity of the branch 45 of the clamp 32.

As well as being adapted to be wound onto a pulley, the belt 64 has a curved section imparting to it a bending resistance enabling it to transmit a thrust force.

The belt 64 crosses the enclosure 30 by means of the window 39 and an oblong hole 46 formed in the small branch 45 of the clamp 32 (figures 2 to 4).

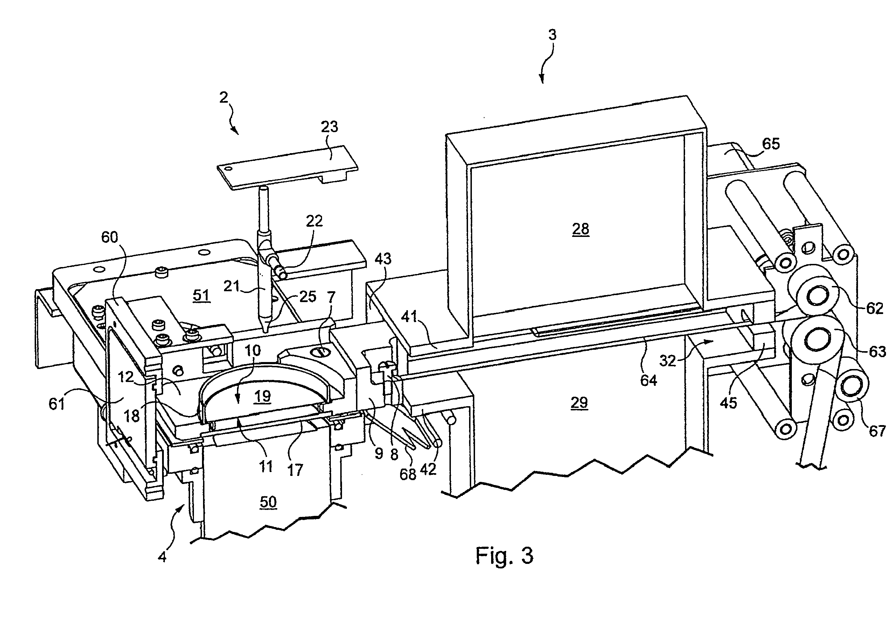

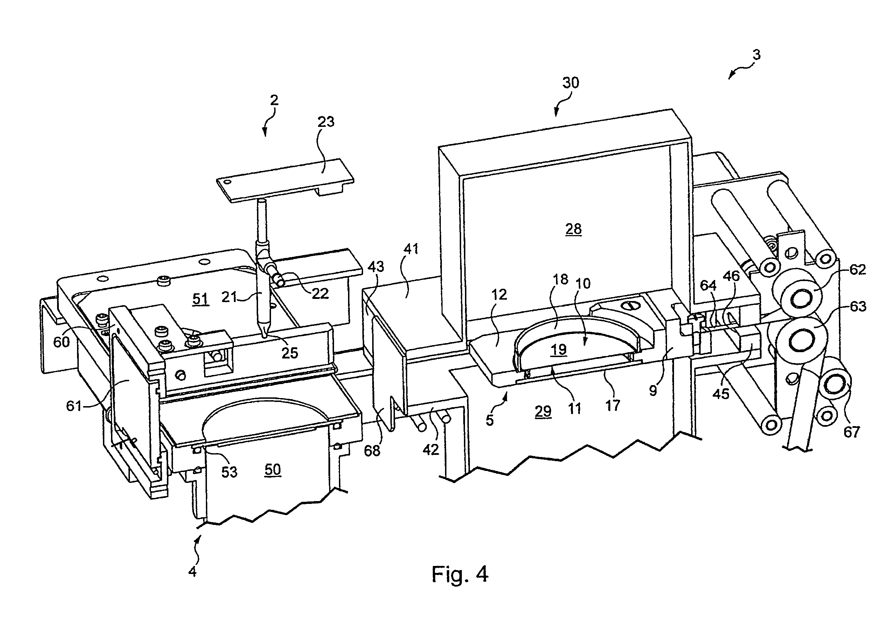

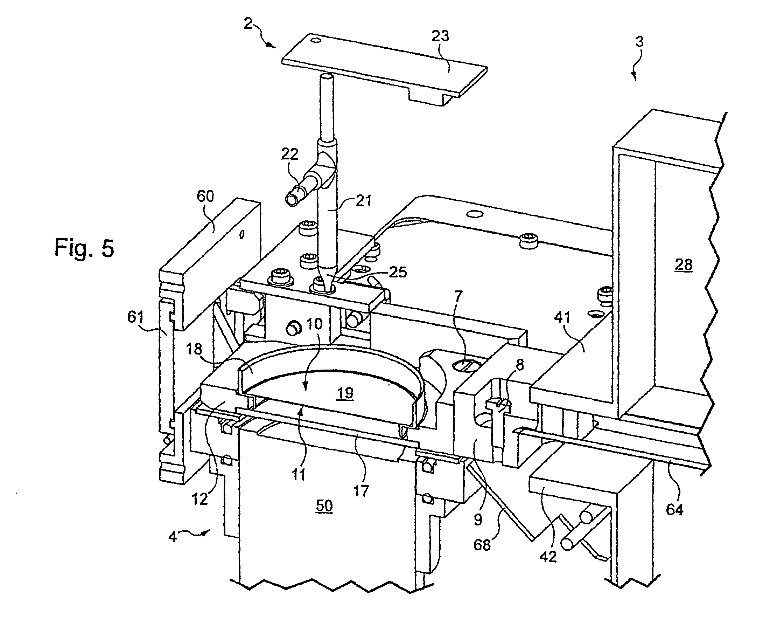

The carriage 5 is fixed to the belt to move the carriage between a receiving position (figures 1 and 2), a first processing position (figures 3, 5 and 6) and a second processing position (figure 4).

The device 1 also includes on the side opposite the motor 65 a window 60 and a cap 61 adapted to render the window 60 light-tight when it is closed.

In the receiving position, with the cap 61 open, the plate 12 projects from the device through the window 60.

In the first processing position, the carriage 5 is between the nozzle 21 and the photomultiplier 50 with the result that, in this position, the spraying station 2 faces and is aligned with the face 10 of the membrane 19, whereas the measuring station 4 faces and is aligned with the face 11 of the membrane through the window 17.

The belt 64 passes completely through the enclosure 30 in the receiving position and in the first processing position.

In the second processing position, the carriage 5 is inside the enclosure 30 of the lysis station 3 so that the membrane 19 is entirely inside this enclosure and the rim 9 is between the flanges 41 and 42.

Sensors at the various processing stations monitor the operating state of the device, in particular an RFID reader/writer beside the flask containing the reagent, a plurality of carriage position sensors and a plurality of temperature sensors (for the photomultiplier and the magnetron, for example).

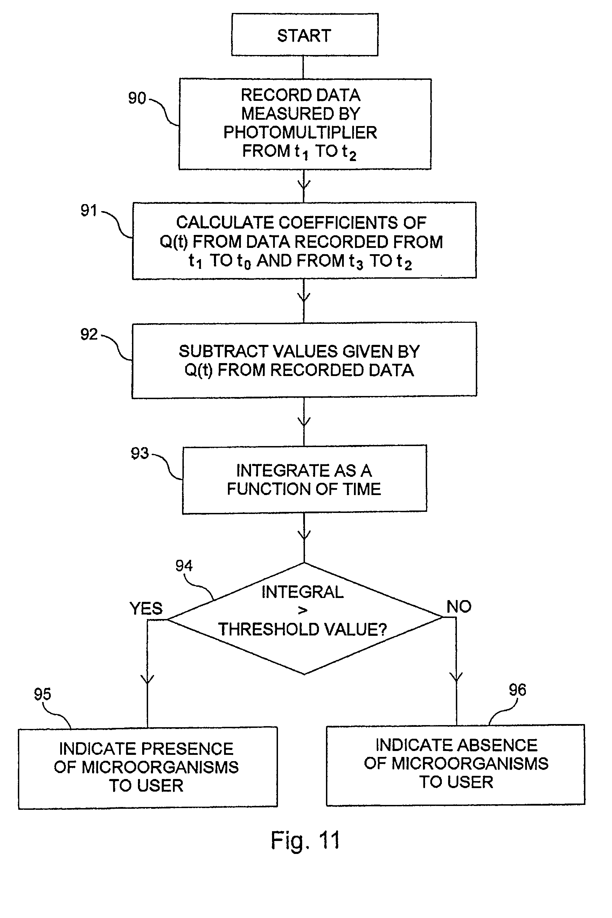

The spraying station 2, the lysis station 3, the measuring station 4, the motors 54 and 65 and the various sensors are connected to a microcomputer 82, as shown in the figure 10 block diagram.

The microcomputer 82 is adapted in particular to execute instructions for launching or stopping an analysis cycle, to receive instructions from an operator via a man-machine interface 85 and to store data coming from the photomultiplier in a memory. The microcomputer 82 is also adapted to display on a screen 83 and/or to print out via a label printer 84 information intended for the operator (for example the progress of the current cycle, the number of cycles still available in the flask, the result of preceding analyses, and various device alarm and maintenance reports).

The operation of the device 1 is described next.

Before carrying out an analysis cycle, the operator collects any microorganisms that may be present in a liquid or a gas or on a surface on the microporous membrane 19 of the filter unit 15.

After selecting the appropriate plate 12 for cooperating with the body 18 of the filter unit and screwing it onto the rim 9, the operator then places the filter unit 15 on the mobile carriage 5 (which at this time is in the receiving position represented in figures 1 and 2), centering it relative to the flange 13, the lower edge of the body 18 of the unit 15 then resting on this flange.

Using a man-machine interface, the operator then commands the launching of a membrane analysis cycle.

In particular, the operator chooses from three different cycles, namely a cycle with no preprocessing, a "manual" preprocessing cycle and an automatic preprocessing cycle.

The various steps of the manual preprocessing cycle are described next.

Initially, the control module runs the motor 65 to turn the pulleys 62, 63 and 67 to move the mobile carriage 5 in translation.

This moves the carriage from its receiving position to its first processing position (figure 3) between the spraying module 2 and the measuring module 4. During this movement, when the whole of the carriage 5 has passed through the window 60, the cap 61 of the device, which is spring-loaded, closes and subsequent steps are carried out in an enclosed and dark environment.

In the first processing position, the station 2 sprays a predetermined volume of reagent. The spraying nozzle 21 is designed to deposit microdroplets of the reagent homogeneously and regularly over the whole of the membrane 19. Spraying microdroplets divides the volume of liquid deposited sufficiently to avoid any risk of dilution.

The reagent is therefore brought into contact with unwanted ATP on the membrane coming not from the microorganisms that it retains but from external contamination, for example during transportation or filtration.

Bringing the reagent into contact with the unwanted ATP causes a chemical reaction that generates light and consumes the unwanted ATP. The unwanted ATP consumed in this way will not interfere with subsequent steps of the analysis cycle.

The reagent does not interact with the ATP of the microorganisms, which at this stage of the cycle is still protected from the reagent by the cell walls of the microorganisms.

When the reagent has been sprayed, the control module runs the motor 65 to move the mobile carriage 5 into the enclosure 30 through the window 39 to take up the second processing position inside the microwave cavity of the lysis station 3.

When the carriage 5 is introduced into the enclosure 30, the flap 68, which is spring-loaded, closes to isolate the enclosure 30 from the remainder of the device and to minimize electromagnetic interference caused by the emission of microwaves.

The magnetron 31 then emits a single-wave field so that an incident wave propagates in the microwave cavity formed by the enclosure 30. Standing waves are established in the enclosure (as a result of reflection of the incident wave by the surfaces of the enclosure 30).

The portions of the flanges 41 and 42 situated in the vicinity of the window 39 and the opening 46 also help to minimize the leakage of magnetic field through these openings.

Excited by the microwave field, the polarized molecules (in particular the water contained in the membrane after filtration and/or resulting from the first spraying of the reagent) heat the membrane 19 to a temperature of about 90°C.

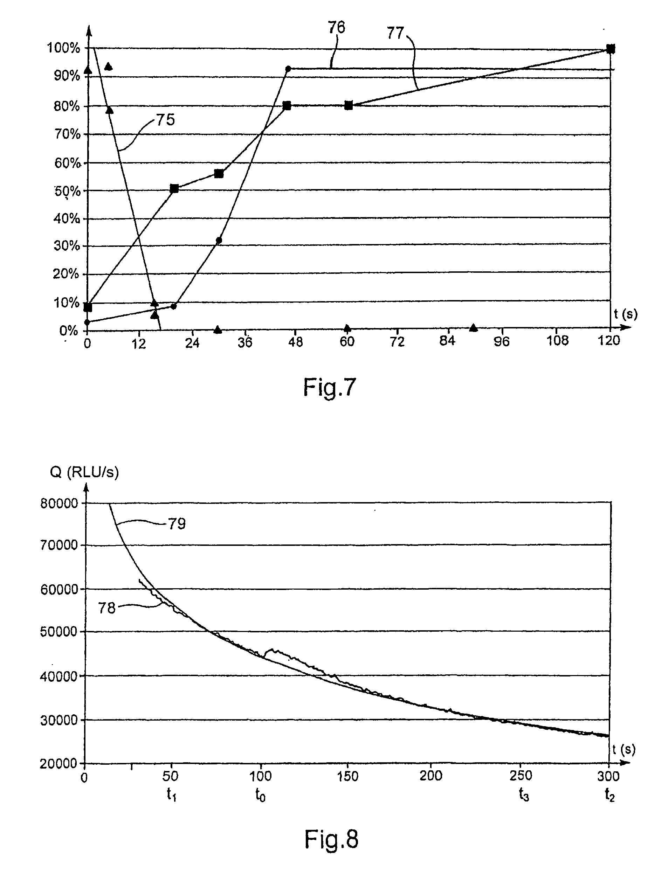

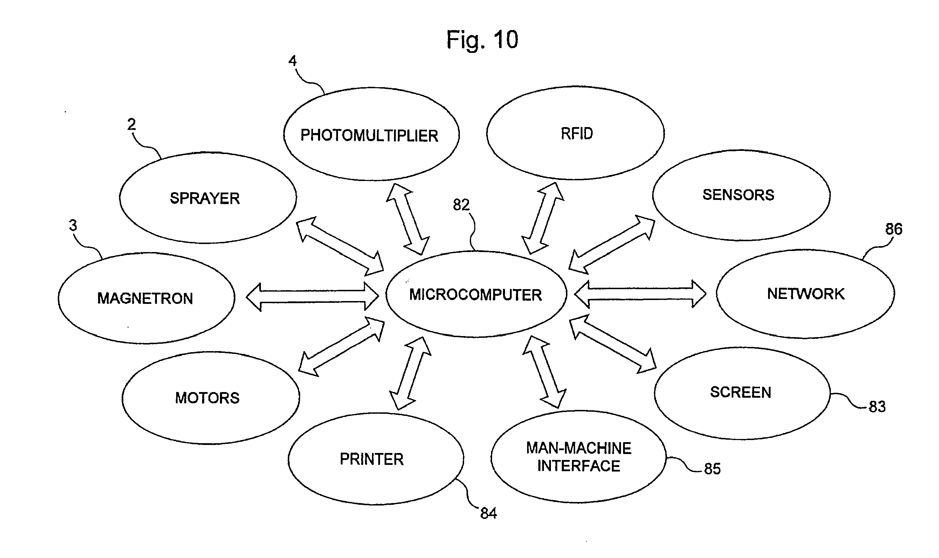

At that temperature, the reagent on the membrane is rapidly eliminated, as shown by the curve 75 in figure 7, which represents as a function of time the proportion of reagent still active (remanent activity level).

After 6 seconds of exposure to this temperature, 20% of the reagent has been eliminated, after 15 seconds 90% of the reagent has been eliminated and after 17 seconds all of the reagent has been eliminated.

The lysis kinetics of the microorganisms are slower at this temperature. The curves 76 and 77 in figure 7 represent the percentage of microorganisms having undergone lysis as a function of time and for two types of microorganism (saccharomyces cerevisae in the case of the curve 76 and cryptococcus in the case of the curve 77). Note that only a small quantity of microorganisms has undergone lysis by the time at which all of the reagent has been eliminated.

Thus the cell walls of most of the microorganisms have not undergone lysis (and the ATP of the microorganisms have therefore not been rendered accessible) by the time the reagent previously deposited has been eliminated. Thus the major portion of the ATP of the microorganisms is not consumed by the reagent.

What is more, elimination of the reagent is accelerated because the increase in temperature leads to partial drying of the membrane 19 making the heat more effective at eliminating the reagent.

Microwave heating represents an input of only the necessary quantity of energy, which is a function of the quantity of water present on the membrane, without producing residual heat that could interfere with subsequent process steps.

After this heating step, the ATP of the microorganisms that have undergone lysis is accessible to be analyzed. The motor 65 is then run to move the carriage 5 out of the enclosure 30 and return it to the first processing position, after which the motor 54 of the closure member 52 of the measuring station is run to turn the crank 55 and move the opaque plate 53 to a position away from the photomultiplier 50 (figure 3).

The photomultiplier then measures the quantity of light that it receives through the window 17 emitted by the materials situated in the "field of view" of the photomultiplier (here the membrane 19 and its immediate surroundings).

The operations carried out by the microcomputer when the closure member has been opened are described next with reference to the figure 11 flowchart.

In the operation 90, from a time t₁ (here 30 s) to a time t₂ (here 300 s), the microcomputer records the measurement data transmitted by the photomultiplier.

At a time t₀ after t₁ and before t₂ (figure 8), the spraying station 2 effects a second regular and homogeneous deposition of microdroplets of the reagent contained in the flask over the whole of the membrane 19, while the recording of the quantity of light continues.

Light is emitted from both sides of the membrane 19 and the plate 12 because of the small thickness of the membrane and the orifice formed in the plate 12 for this purpose.

The quantity of light recorded in relative light units (RLU) as a function of time is indicated by the curve 78 in figure 8, which shows that the quantity of light decreases regularly and logarithmically to the time t₀ corresponding to the time at which the reagent is sprayed onto the membrane.

This decreasing phase corresponds to the de-excitation by natural phosphorescent emission of photons by the material situated in the field of view of the photomultiplier.

Beyond the time t₀ there occurs an abrupt transition in the emission of light because of the reagent coming into contact with the ATP of the microorganisms that has been rendered accessible by the lysis step.

After this abrupt transition, the quantity of light again decreases logarithmically.

Using the recorded data, the microcomputer performs the operation 91 and extrapolates as a function of time the quantity of light that would have been emitted if the reagent had not been deposited on the membrane.

In the present example, this operation is based on the data recorded from t₁ (50 s) to t₀ (100 s) and from data recorded from t₃ = t₀ + x to t₂ (300 s), where x is chosen so that t₃ is sufficiently far away from t₀ for it to be considered that beyond t₃ the influence of adding the reagent becomes negligible (here t₃ = 250 s). The microcomputer deduces from the recorded data the coefficients of a logarithmic function varying globally from t₁ to t₀ and from t₃ to t₂ as the quantity of light represented by the curve 78.

In the present example, this function is Q(t) = -16518.ln(t) + 120334 and is represented by the curve 79 in figure 8; here the coefficient of correlation between the recorded data and the data from the extrapolated function is 0.09994 in the time intervals [t₁, t₀] and [t₃, t₂].

The curves 78 and 79 are significantly different during a time interval whose lower limit is the time t₀ (100 s) at which the reagent was sprayed (here the interval [100 s, 150 s]).

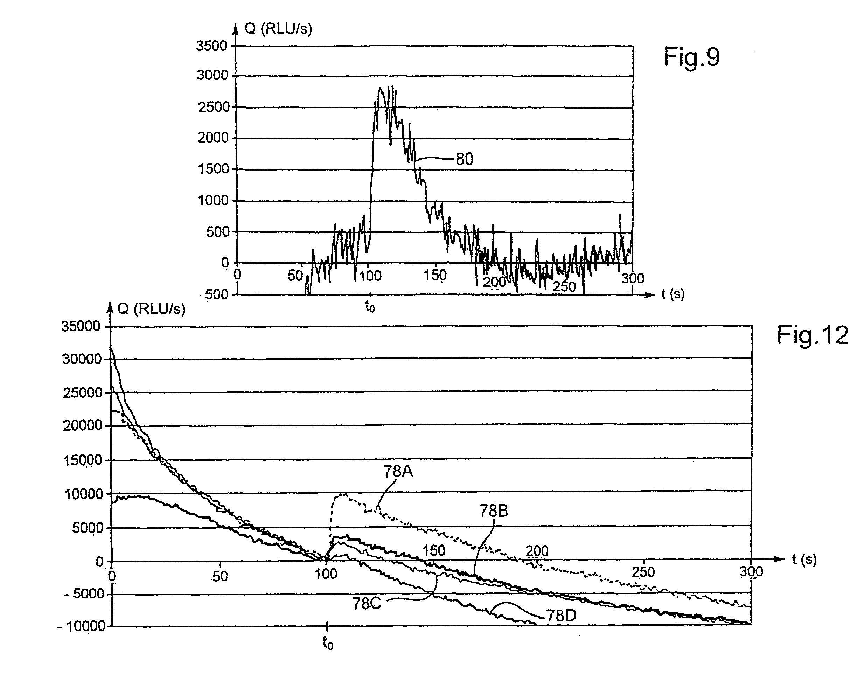

By means of the operation 92, the microcomputer then determines from the recorded and extrapolated quantities of light respectively represented by the curves 78 and 79 a set of values as a function of time representative of the quantity of light coming only from the ATP of the microorganisms coming into contact with the reagent. That set of values is represented by the curve 80 in figure 9.

In the present example, for each time from t₁ to t₂ at which a quantity of light value has been recorded, the microcomputer calculates the difference between that value and the value for the same time given by the extrapolation function Q(t).

This point-by-point subtraction digitally filters unwanted luminous background noise ("decreasing white") to obtain the set of values corresponding to the quantity of light coming only from the ATP of the microorganisms.

Filtering the background noise eliminates the effects of the storage conditions (the influence of which is important for the emission of natural light), the nature of the product deposited or filtered on the membrane (emitting light itself depending its composition), and the experimental analysis conditions (light-tightness, thermal interference, efficiency of the photomultiplier).

The quantity of light filtered in this way is then integrated from time t₁ to time t₂ (operation 93) and a test (94) compares the results of this integration to a threshold corresponding to the microorganism detection sensitivity required by the user.

Among other things, the value of this threshold is chosen as a function of the type of microorganism to be detected, as the mass of ATP contained in the microorganisms can vary significantly from one type of microorganism to another.

If the result of this integration is above the threshold value, the ATP giving rise to this light is deemed to be caused by the presence of microorganisms on the membrane.

Conversely, if the result of this integration is below the threshold value, ATP is deemed not to be present in sufficient quantities to consider that microorganisms are present (or present in sufficiently large quantities) on the membrane.

The microcomputer then displays the appropriate information on the screen of the device by carrying out either the operation 95 or the operation 96, as a function of the result of the test 94.

Once the analysis has been carried out the control module moves the carriage 5 from the first processing position to the receiving position, in which the operator can remove the filter unit 15 that has been analyzed and replace it with the next unit to be analyzed.

The disposition of the measuring station 4 and the spraying station 2 relative to the membrane 19 in the first processing position means that the photomultiplier 50 is as close as possible to the membrane 19 and disposed transversely to it, without being impeded by the spraying station and without impeding the spraying station. This arrangement collects the maximum light and therefore optimizes the detection sensitivity of the photomultiplier.

Moreover, using a closure member having a thin opaque plate and a remote opening and closing mechanism further reduces the distance between the photomultiplier 50 and the membrane 19 and thus increases detection sensitivity.

The configuration of the device, the preprocessing step and the data processing of the recorded light signal make the detection device highly sensitive.

A device of the above kind can detect the presence of only around ten femtograms of ATP.

This sensitivity means that a wide variety of microorganisms can be detected and the presence of a single microorganism on the membrane can be detected without incubation.

The preprocessing steps carried out in the case of an automatic cycle are described next.

The automatic analysis cycle ensures that all the unwanted ATP has been consumed.

In figure 12, which represents the quantities of light recorded before and after depositing a reagent at the time t₀ for various quantities of unwanted ATP that have not been consumed (170 fg in the case of the curve 78A, 66 fg in the case of the curve 78B, 52 fg in the case of the curve 78C and 18 fg in the case of the curve 78D), the unwanted ATP that has not been consumed may be present in large quantities and cause an erroneous analysis (detection of "false positives": the presence of microorganisms is detected although the membrane does not contain any) .

It is therefore beneficial to provide the user with a cycle which confirms that the preprocessing step has consumed all of the unwanted ATP.

Before effecting the lysis of the microorganisms, with the carriage 5 in the first processing position and at the same time as spraying the reagent, the closure member 52 is opened in order for the photomultiplier 50 to measure continuously the quantity of light emitted by the membrane and its immediate surroundings during the spraying step. The microcomputer compares this measurement to a predetermined threshold value and spraying of the reagent continues for as long as the quantity of light measured by the photomultiplier is above that threshold.

When the quantity of light measured falls below the threshold, the unconsumed mass of unwanted ATP still present on the membrane is deemed to have become negligible and spraying is stopped. The carriage is then moved to the second processing position to continue the analysis cycle.

In a variant, instead of integrating all of the values corresponding to the quantity of light represented by the curve 80, the microcomputer can select the maximum value from that set and compare it to a predetermined threshold value.

A further variant is for the membrane to be heated by infrared means.

A further variant is for the step of thermolysis to be replaced by a step of spraying a reagent either achieving chemical lysis of the microorganisms or rendering the membrane permeable to the microorganisms in order to make the ATP that they contain accessible to the reagent for revealing ATP by luminescence that is sprayed subsequently. A further variant is to use the device to count the microorganisms on the membrane either by substituting a CCD camera for the photomultiplier or by processing and/or analyzing the membrane several times, one elementary region at a time.

In still another variant, the result obtained by calculation of the area by integration or of the maximum value may also be confirmed by the analysis of other additional criteria making it possible to ensure that the result obtained is not distorted by extraneous disturbance.

This analysis consists in the study of the characteristics of the light quantity measured between t₁ and t₂ and represented by the curve 78 in Figure 8.

It is thus possible to study one, several or all of the criteria set forth below.

A first criterion consists of verifying that the quantity of light beyond the time t₀ decreases (negative slope). This is because, if the slope is positive, this means that the quantity of light is continuing to increase after the abrupt transition of light induced by the spraying of the reagent, which is in contradiction with the fact that the quantity of light decreases during the consumption of the reagent that has just been sprayed (see curve 78 between t₀ and t₂) . This phenomenon thus derives in this case not from the presence of microorganisms in contact with the reagent inducing the luminescence, but from an extraneous phenomenon (false positive). What is meant by false positive is the results erroneously indicating the presence of microorganisms on the membrane.

A second criterion consists of verifying that the amplitude of the abrupt transition of light on deposition of the reagent is greater than a predetermined threshold value (for example 100 RLU) in order to ensure that the jump in light induced by the spraying is sufficiently significant and thus indeed arises from the placing of the reagent in contact with the microorganisms on the membrane.

A third criterion consists of verifying that the quantity of light remains greater than a predetermined value (for example 100 RLU) at a predetermined time after the deposition of the reagent (here t₀ + 50s) with respect to the quantity of light before the deposition of the reagent in order to ensure that the jump in light generated by the placing of the reagent in contact with the membrane remains significant, even at a time more distant from the spraying. This criterion thus makes it possible not to keep the "peaks" of light in the positive results that apparently have only a small temporal width and which are therefore apparently not due to the presence of microorganisms but are generated for example solely by the simple passage of the membrane from a dry to a wet state.

A fourth criterion consists of verifying that the quantity of light emitted by the support after the deposition of the reagent decreases differently from the quantity of light emitted by the support before that deposition.

This is because, if the quantity of light decreases in identical manner (with the same slope) before and after spraying (the abrupt transition being the only difference), this means that this decrease is only linked to a simple de-excitation of the membrane subjected to the light of the environment but is not caused by the presence of microorganisms on the membrane placed in contact with the sprayed reagent (false positive).

A fifth criterion consists of verifying that the time during which the signal obtained after spraying stays above a predetermined threshold (for example 100 RLU) is greater than a predetermined time (for example 10s) in order not to keep the peaks of light as positives that are apparently not caused by the presence of microorganisms on the membrane.

These criteria may be verified independently from each other, each making it possible to confirm or refute that the signal emitted in response to the spraying of the reagent is due to the presence of microorganisms.

Whenever one of these tests is negative, the microcomputer displays information on the screen of the device enabling the user to be warned that the analysis of the support leads to the conclusion that the result obtained is a false positive.

In a variant not shown the recording has not taken place continuously between t₁ and t₂, but is suspended for example for a short time centered around t₀ (for example in order to enable the movement of a carriage in the case in which the spraying and measuring stations do not face each other but are beside each other) and/or the extrapolation is only carried out on the basis of the data recorded before the spraying of the reagent (those acquired between t₁ and t₀).

Should the recording be interrupted in the neighborhood of t₀, the extrapolated curve is used to determine the value of light quantity just before the spraying of the reagent (which takes place at t₀) in order to be able to determine the amplitude of the light jump despite the absence of recording just before t₀.

The present invention is not limited to the embodiments described and shown and encompasses any variant execution thereof.

## Claims

1. Method of fast microbiological analysis of a support (19) adapted to retain microorganisms, including the step of selecting a reagent adapted to reveal the presence of ATP by luminescence and:
- the step of acting on said microorganisms to render the ATP of said microorganisms accessible to said reagent; then
- the step of depositing on said support (19) said reagent; and
- the step of detecting the response of said support (19) to said reagent;
**characterized in that** said detection step includes:
- the step (90) of recording the quantity of light emitted by said support (19) as a function of time from a time t₁ before a time t₀ at which said deposition step is carried out to a time t₂ after said time t₀;
- the step (91) of extrapolating, from said recorded quantity of light, to the quantity of light as a function of time that would have been emitted by said support (19) between said time t₁ and said time t₂ without the deposition of said reagent; and
- the step (92) of determining from said recorded quantity of light and from said extrapolated quantity of light a set of values representative of the quantity of light emitted as a function of time starting from said time t₀ only as a result of bringing said reagent into contact with the ATP.

2. Method according to claim 1, **characterized in that** said step of determining said set of values includes the step (92) of determining the difference between said recorded quantity of light and said extrapolated quantity of light.

3. Method according to either claim 1 or claim 2, **characterized in that**, after the step of determining said set of values, the detection step further includes:
- the step (93) of integrating said set of values as a function of time; and
- the step (94) of comparing the result of that integration to a predetermined value to deduce the presence of microorganisms.

4. Method according to either claim 1 or claim 2, **characterized in that**, after the step of determining said set of values, the detection step further comprises:
- the step of selecting the maximum value of said set of values;
- the step of comparing the result of that selection to a predetermined value to deduce the presence of microorganisms.

5. Method according to any one of claims 1 to 4, **characterized in that** the detection step further comprises a step of discriminating false positives comprising a step of implementing at least one of the following tests:
- verifying that said quantity of light emitted by said support (19) after the time t₀ decreases over time;
- verifying that the amplitude of the abrupt transition of light caused by the deposition of the reagent is greater than a predetermined value;
- verifying that the quantity of light remains greater than a predetermined value at a predetermined time after said deposition of the reagent with respect to the quantity of light before the deposition of the reagent;
- verifying that the decrease in the quantity of light emitted by said support (19) after said deposition of the reagent is different from the decrease in the quantity of light emitted by said support before said deposition, and/or
- verifying that the time during which the quantity of light emitted by said support (19) is greater than a predetermined threshold is greater than a predetermined time;
then after the step of implementing at least one of the preceding tests, the step of indicating the presence of a false positive if the result of at least one of the tests is negative.

6. Method according to any one of claims 1 to 5, **characterized in that** the recording step includes:
- the step of selecting a photomultiplier (50) ;
- the step of placing said support (19) facing said photomultiplier (50);
- the step of opening at said time t₁ a closure member (52) disposed in front of said photomultiplier (50);
- the step of measuring the quantity of light received by said photomultiplier (50) between said time t₁ and said time t₂; and
- the step of storing said measurement in a memory.

7. Method according to any one of claims 1 to 6, **characterized in that** said extrapolation step is effected by calculating the coefficients of a logarithmic function decreasing as a function of time and varying as the quantity of light that would be emitted by said support without the deposition of said reagent would vary.

8. Method according to any one of claims 1 to 7, **characterized in that** said extrapolation step is based on said quantity of light recorded between said time t₁ and said time to.

9. Method according to any one of claims 1 to 8, **characterized in that** said extrapolation step is based on said quantity of light recorded between a time t₀+x and said time t₂, where x is a predetermined duration.

10. Method according to any one of claims 1 to 9, **characterized in that** said reagent for revealing the presence of ATP by luminescence is a reagent based on luciferin-luciferase.

11. Method according to any one of claims 1 to 10, **characterized in that** said step of acting on said microorganisms includes a step of lysis of said microorganisms.

12. Method according to claim 11, **characterized in that** said lysis step is effected by heating said support (19) .

13. Method according to claim 12, **characterized in that** said heating step includes the step of raising the heating temperature to a value from 50°C to 150°C.

14. Method according to claim 13, **characterized in that** said heating step includes the step of raising the heating temperature to a value from 70°C to 100°C.

15. Method according to any one of claims 12 to 14, **characterized in that** said support (19) is heated by microwaves.

16. Method according to any one of claims 1 to 10, **characterized in that** said step of acting on said microorganisms includes the step of rendering said microorganisms permeable.

17. Method according to claim 16, **characterized in that** said step of rendering said microorganisms permeable includes the step of spraying onto said support (19) a reagent adapted to render said microorganisms permeable.

18. Method according to any one of claims 1 to 17, **characterized in that** said support is a microporous membrane (19).
